# EUROPÄISCHE PATENTSCHRIFT

(11) **EP 3 344 270 B1**
(45) Veröffentlichungstag und Bekanntmachung des Hinweises auf die Patenterteilung: **25.11.2020**
(21) Anmeldenummer: 16758171.9
(22) Anmeldetag: 31.08.2016
(51) Int. Cl.: A61K 36/28, A61K 36/286, A61K 31/201, A61K 31/231, A61K 9/00, A61K 8/37, A61K 8/97, A61K 47/14, A61K 9/06, A61Q 19/00

(54) **ZUSAMMENSETZUNG ENTHALTEND EINEN EXTRAKT AUS ECHINACEA UND LINOLSÄUREDERIVATE**
COMPOSITION CONTAINING ECHINACEA EXTRACT AND LINOLEIC ACID DERIVATIVES
COMPOSITION CONTENANT UN EXTRAIT D'ECHINACEA ET DES DÉRIVÉS D'ACIDE LINOLÉIQUE

(30) Priorität: 31.08.2015 DE 102015011132
(43) Veröffentlichungstag der Anmeldung: 11.07.2018
(73) Patentinhaber: Dr. August Wolff GmbH & Co. KG Arzneimittel, 33611 Bielefeld (DE)
(72) Erfinder: SOEBERDT, Michael, 33611 Bielefeld (DE); ABELS, Christoph, 33611 Bielefeld (DE); KNIE, Ulrich, 33611 Bielefeld (DE)
(74) Vertreter: Ter Meer Steinmeister & Partner
(86) Internationale Anmeldenummer: PCT/EP2016/070438
(87) Internationale Veröffentlichungsnummer: WO 2017/037075

(56) Entgegenhaltungen:
- WO-A1-2012/013551
- US-A1- 2009 197 974
- N.N.: "Echinacea purpurea, Planta tota 10% Salbe", , 1. Dezember 2005 (2005-12-01), XP055311880, Gefunden im Internet: URL:https://www.medicaria.de/images/ecomme rce/01/62/01627942_1970-01_de_s.pdf [gefunden am 2016-10-18]
- SOEBERDT M ET AL: "Anti-inflammatory activity of alkylamides from Echinacea purpurea in keratinocytes in vitro and in mouse models of inflammatory skin diseases", JOURNAL OF INVESTIGATIVE DERMATOLOGY, NATURE PUBLISHING GROUP, US, Bd. 134, Nr. Suppl. 1, 1. Mai 2014 (2014-05-01), Seite S14, XP009192153, ISSN: 0022-202X
- NOTARNICOLA ANGELA ET AL: "Comparison of shock wave therapy and nutraceutical composed of Echinacea angustifolia, alpha lipoic acid, conjugated linoleic acid and quercetin (perinerv) in patients with carpal tunnel syndrome", INTERNATIONAL JOURNAL OF IMMUNOPATHOLOGY AND PHARMACOLOGY, SAGE PUBLICATIONS LTD, GB, Bd. 28, Nr. 2, 1. Juni 2015 (2015-06-01), Seiten 256-262, XP009192144, ISSN: 0394-6320, DOI: 10.1177/0394632015584501
- HOLCOVA S ET AL: "Effects of an Echinacea ointment on the regeneration of irritated skin", KOSMETISCHE MEDIZIN 2004 DE, Bd. 25, Nr. 5-6, 1. Januar 2004 (2004-01-01), Seiten 247-251, XP9192133, ISSN: 1430-4031

## Beschreibung

Die vorliegende Erfindung betrifft pharmazeutische und kosmetische Zusammensetzungen aus einem Extrakt aus *Echinacea* und Linolsäurederivaten, insbesondere Emulsionen enthaltend einen Extrakt aus *Echinacea* und Linolsäurederivaten als Wirkstoffkombination, sowie die Verwendung solcher Zusammensetzungen als Arzneimittel, Medizinprodukt und/oder Kosmetikum.

### Stand der Technik

Präparate des purpurnen Sonnenhutes (*Echinacea purpurea* und *Echinacea angustifolia*) sind bei der Behandlung von Erkältungen und Infekten der oberen Atmungsorgane weit verbreitet. Dabei wird generell angenommen, dass die Wirkung von *Echinacea* durch Interaktionen mit dem Immunsystem ermöglicht wird. Es konnte gezeigt werden, dass ein bestimmter Anteil in den Extrakten aus *Echinacea,* dessen entzündungshemmende Wirkung bereits von anderen Arbeitsgruppen beschrieben wurde, das endogene Cannabinoidsystem beeinflusst. Bei diesem Anteil handelt es sich um die Stoffklasse der Alkylamide (Raduner S et al., JBiol Chem 2006, 281, 14192-14206). Diese Alkylamide zeigen eine immunmodulatorische Aktivität (Gertsch J, Planta Med 2008, 74, 638-650).

Capsaicin ist ein Agonist des Transient Receptor Potential Vanilloid 1 (TRPV1), der nach topischer Verabreichung antinozizeptive und antipruritische Effekte zeigt. Topische Darreichungsformen von Capsaicin werden u.a. für die Behandlung von Hämodialyse induziertem (Breneman DL, et al. JAm Acad Dermatol 1992, 26, 91-94), aquagenen (Lotti T, et al. J Am Acad Dermatol 1994, 30, 232-235) und brachioradialem Juckreiz (Goodless DR, Eaglestein WH, J Am Acad Dermatol 1993, 29, 783-784) eingesetzt. Weitere Einsatzgebiete sind Notalgia paraesthetica (Leibsohn E, Cutis 1992, 49, 335-336) und Prurigo nodularis (Ständer S, Luger T, Metze D, J Am Acad Dermatol 2001, 44, 471-478).

Bestandteile aus *Echinacea purpurea* wurden als Inhibitoren der Tyrosinase beschrieben (Jiang L, et al., J Med Plants Res 2012, 6, 5317-5321). Die Aktivität der Tyrosinase steht im Zusammenhang mit der Produktion von Melanin durch Melanozyten (Kim, YJ, Uyama H, Cell Mol Life Sci 2005 62, 1707-23). Die Inhibition der Tyrosinase kann also zu einer verringerten Biosynthese von Melanin führen, was wiederum zu einer verminderten Pigmentierung z.B. der Haut führen kann. Dies kann zu kosmetischen Beeinträchtigungen bei der Verwendung von Darreichungen führen, die einen Tyrosinase-Inhibitor, wie zum Beispiel einen *Echinacea-Extrakt,* enthalten, da es zu Unterschieden in der Pigmentierung von behandelter und unbehandelter Haut kommen könnte.

Die Verbesserung des Hautzustands nach Behandlung mit einer Formulierung mit einem *Echinacea*-Extrakt wurde beschrieben (Yotsawimonwat S, et al., Int J Cosm Sci 2010, 32, 340-346). Hierfür wurden alkoholische Extrakte aus den oberirdischen Pflanzenteilen von *Echinacea purpurea* eingesetzt. Dabei konnte gezeigt werden, dass sowohl eine Creme- als auch eine Gelformulierung, die den alkoholischen Extrakt aus *Echinacea* enthalten, die Hydratisierung der Haut in Probanden ohne Barriereschädigung verbessern. Die Lagerfähigkeit der Zubereitungen war allerdings stark eingeschränkt. Sie betrug bei 4°C 2 bzw. 4 Monate für die Creme und das Gel. Durch Zugabe eines Antioxidans konnte die Haltbarkeit auf 7 Monate erhöht werden.

Neben den bekannten immunstimulatorischen und antientzündlichen Effekte von Extrakten aus *Echinacea purpurea* gibt es kontroverse Befunde aus klinischen Studien bezüglich der Nebenwirkungen mit Präparaten, die *Echinacea* enthalten (Manayi A, Varizian M, Saeidnia S, Pharmacogn Rev 2015, 9, 63-72). So wurden u.a. Pruritus, Erytheme und Urtikaria als schwere Nebenwirkungen bei der Behandlung mit *Echinacea* festgestellt.

WO 2012/013551 A1 offenbart einen Extrakt aus *Echinacea angustifolia* in Kombination mit Leinsamen-, Oliven- oder Nachtkerzensamenöl.

Im Artikel "Echinacea pupurea, Planta tota 10% Salbe", 1. Dezember 2005 wird eine Kombination von *Echinacea purpurea* mit Sesamöl offenbart.

US 2009/197974 A1 beschreibt die Kombination von Safloröl und *Echinacea angustifolia.*

Der Artikel "Anti-inflammatory activity of alkylamides from Echinacea purpurea ...", Soeberdt M. et al., Journal of Investigative Dermatology, Nature Publishing Group, US, Bd. 134, Nr. Suppl. 1, 1. Mai 2014, beschreibt die anti-entzündliche Wirkung von Alkylamiden aus *Echinacea purpurea.*

Der Artikel "Comparison of shock wave therapy and nutraceutical composed of Echinacea angustifolia, alpha lipoic acid ..." Notarnicola, Angela et al., International Journal of Immunopathology and Pharmacology, Sage Publications Ltd., GB, Bd. 28, Nr. 2, 1. Juni 2015, Seiten 256-262, beschreibt die Verwendung von *Echinacea angustifolia* in einem Nutraceutical zur Behandlung von CTS (Carpal Tunnel Syndrom).

Der Artikel "Effects of an Echinacea ointment on the regeneration of irritated skin", Holcova S. et al., Kosmetische Medizin 2004 DE, Band 25, Nr. 5-6, 1. Januar 2004, Seiten 247-251, beschreibt die Effekte einer Echinacea-Salbe auf die Regeneration belasteter und irritierter Haut.

### Aufgabenstellung

Es bestand die Aufgabe, eine Wirkstoffkombination zur Verfügung zu stellen, die im Vergleich zu einem Extrakt aus *Echinacea* allein verbesserte Eigenschaften besitzt. Insbesondere sollte eine Zusammensetzung zur Verfügung gestellt werden, die eine antipruritische Wirkung aufweist. Ferner bestand die Aufgabe darin, die Stabilität der Formulierung zu verbessern, um eine kommerzielle Anwendung der Zusammensetzung zu ermöglichen. Eine weitere Aufgabe der vorliegenden Erfindung war es, unerwünschte Wirkungen des Extrakts aus *Echinacea,* die eine kosmetische Anwendung beeinträchtigen, zu verhindern. Insbesondere sollte die Bildung von Erythemen nach Behandlung mit dem Extrakt aus *Echinacea* verhindert werden. Von besonderem Interesse war es weiterhin, einen negativen Einfluss des Extrakts aus *Echinacea* auf die Pigmentbildung in der Haut zu verhindern.

Diese Aufgaben werden durch die vorliegende Anmeldung gemäß den Ansprüchen gelöst. Insbesondere hat sich überraschenderweise gezeigt, dass die erfindungsgemäße Zusammensetzung eine ausgezeichnete antipruritische Wirkung, beispielsweise bei Patienten mit atopischer Dermatitis, aufweist und gleichzeitig eine hohe Haltbarkeitsdauer von wenigstens zwei Jahren bei Raumtemperatur zeigt. Dadurch ist eine kommerzielle Vermarktung sehr gut möglich. Darüber hinaus hat sich erfindungsgemäß gezeigt, dass die Regeneration der natürlichen Hautbarriere in Probanden und Patienten mit bereits gestörter Hautbarriere sehr effektiv gefördert wird. Überraschend war auch, dass die Behandlung mit den erfindungsgemäßen Zusammensetzungen zu einer signifikanten Erhöhung der Lipide in der Haut führt. Erwähnenswert ist in diesem Zusammenhang insbesondere die Erhöhung des langkettigen Ceramids EOS, welches für die Stabilität der Stratum corneum-Lipiddoppelschichten besonders wichtig ist, da hier eine ω-Hydroxy-Fettsäure im Ceramidgrundkörper mit Linolsäure verestert ist. Durch die Veresterung entsteht nämlich eine C₃₆-Seitenkette, die bis in die nächste Lipiddoppelschicht hineinragt. Daher spricht man auch von der "Nagelfunktion" dieser Ceramide.

Belegt wird die antipruritische Wirkung unter anderem durch eine überraschend deutliche Aktivierung des Transient Receptor Potential Vanilloid 1 (TRPV1) bei Verabreichung der erfindungsgemäßen Wirkstoffkombination. Ferner war es für den Fachmann nicht zu erwarten, dass die Kombination der Wirkstoffe *Echinacea* und Linolsäure/Linolsäurederivate den von *Echinacea* bekannten unerwünschten Effekt auf die Pigmentbildung der Haut verhindert. Die bei Verabreichung von *Echinacea* auf der Haut zu beobachtende Störung der Pigmentbildung (verminderte Pigmentierung) tritt bei der erfindungsgemäßen Wirkstoffkombination von *Echinacea* mit Linolsäure und/oder Linolsäurederivaten nicht auf. Belegt wird dieses durch die überraschende unverminderte Tyrosinase-Aktivität bei Verwendung der erfindungsgemäßen Wirkstoffkombination.

Die vorliegende Erfindung betrifft somit eine Zusammensetzung enthaltend a) einen Extrakt aus *Echinacea* und b) Linolsäure und/oder Linolsäurederivate wie in Anspruch 1 definiert. Bevorzugt ist der Extrakt aus *Echinacea* ein lipophiler Extrakt. Besonders bevorzugt ist der Extrakt aus *Echinacea* ein Extrakt, der mit überkritischem CO₂ gewonnen wird. Erfindungsgemäß bevorzugt ist zudem ein Alkylamidgehalt des Extrakts zwischen 1 und 50%, bevorzugt zwischen 10 und 40% und besonders bevorzugt zwischen 15 und 30% (gewichtsbezogen).

Erfindungsgemäß enthält die Zusammensetzung die Bestandteile a) und b) in den Gewichtsverhältnissen 1:1000 bis 1:1; bevorzugt 1:50 bis 1:10. Ferner enthält die Zusammensetzung der Erfindung bevorzugt 0,001 - 5 Gew.% *Echinacea*-Extrakt, bevorzugt 0,025 - 2 Gew.% *Echinacea*-Extrakt, besonders bevorzugt 0,05 - 1 Gew.% *Echinacea-*Extrakt, bezogen auf das Gewicht der Gesamtzusammensetzung, und 0,001-10 Gew.% Linolsäure und/oder eine derartige Menge an Linolsäurederivaten, die 0,001-10 Gew.% Linolsäure entsprechen, bevorzugt 0,01-5 Gew.% Linolsäure und/oder eine derartige Menge an Linolsäurederivaten, die 0,01-5 Gew.% Linolsäure entsprechen, jeweils bezogen auf das Gewicht der Gesamtzusammensetzung.

Die erfindungsgemäße Zusammensetzung enthält bevorzugt weiterhin einen oder mehrere kosmetische und/oder pharmazeutische Hilfsstoffe. Besonders bevorzugt ist die Zusammensetzung eine Emulsion.

Erfindungsgemäß wird die Zusammensetzung als Arzneimittel, als Medizinprodukt oder als Kosmetikum verwendet. Dabei dient sie bevorzugt zur Behandlung von Juckreiz, trockener Haut und/oder irritierter Haut (z.B. geröteter Haut nach Sonnenbrand, Abschürfungen oder anderweitig gereizter Haut etc.), aber auch von entzündlichen Zuständen der Haut, wie insbesondere atopischer Dermatitis (Neurodermitis, atopisches Ekzem). In einer bevorzugten Ausführungsform ist die Haut die Kopfhaut.

Nachstehend werden die einzelnen Bestandteile der erfindungsgemäßen Zusammensetzung näher erläutert.

Der erfindungsgemäß verwendete *Echinacea*-Extrakt als Bestandteil a) der Zusammensetzung ist aus *Echinacea purpurea*, wobei der Extrakt aus den Wurzeln (Radix) gewonnen wird. Die Extraktion kann erfindungsgemäß mit organischen Lösungsmitteln, wie Alkoholen (z.B. Methanol, Ethanol), wässrigen Lösungen von Alkoholen (z.B. Methanol, Ethanol), Alkanen (z.B. Pentan, Hexan, Heptan), Chlorkohlenwasserstoffen (z.B. Chloroform, Methylenchlorid), Ketonen (z.B. Methylethylketon, Aceton), oder mittels Extraktion mit überkritischem Kohlendioxid (CO₂) erfolgen. Lipophile Extrakte sind dabei bevorzugt. Die Extraktion mit überkritischem Kohlendioxid ist erfindungsgemäß besonders bevorzugt, da natürliche Quellkohlensäure eingesetzt werden kann und sich der Extrakt schonend und besonders rein gewinnen lässt. Zudem kann das Lösungsmittel CO₂ leicht rückstandslos entfernt und in einem geschlossenen Kreislaufsystem recycelt werden.

Der Alkylamidgehalt im Extrakt liegt bevorzugt zwischen 1 und 50%, bevorzugt zwischen 10 und 40% und besonders bevorzugt zwischen 15 und 30% (gewichtsbezogen). Die Bestimmung des Alkylamidgehaltes kann dabei nach bekannten Analysemethoden erfolgen. Beispielhaft genannt sei die massenspektrometrische Bestimmung des Alkylamidgehaltes. Weiterhin kann die Bestimmung des Alkylamidgehalts chromatographisch, z.B. mittels HPLC-Chromatographie, mit Referenzsubstanzen durchgeführt werden.

Als zweiter Wirkstoffbestandteil b) der erfindungsgemäßen Zusammensetzung ist Linolsäure und/oder ein oder mehrere Linolsäurederivat(e) enthalten. Bei Linolsäure handelt es sich um (*cis*,*cis*)-Octadeca-9,12-diensäure, eine zweifach ungesättigte Fettsäure mit 18 Kohlenstoffatomen (18:2). Sie gehört somit zu den Omega-6-Fettsäuren. Erfindungsgemäß kann sowohl die freie Linolsäure als auch ein Linolsäurederivat verwendet werden. Erfindungsgemäß sind Distelöl (Safloröl), Weizenkeimöl und Rapsöl. Linolsäure und Linolsäurederivate können aus diesen Ölen vor Verwendung in bekannter Weise extrahiert werden. Alternativ können die natürlichen Öle und Fette direkt als solche als Linolsäurebestandteil b) in der erfindungsgemäßen Zusammensetzung eingesetzt werden. Distelöl ist dabei besonders bevorzugt.

Die Mengenverhältnisse der Bestandteile a) und b) in der Zusammensetzung lauten bevorzugt wie folgt: 0,001 - 5 Gew.% *Echinacea*-Extrakt, bevorzugt 0,025 - 2 Gew.% *Echinacea-*Extrakt, besonders bevorzugt 0,05 - 1 Gew.% *Echinacea*-Extrakt, bezogen auf das Gewicht der Gesamtzusammensetzung, und 0,001-10 Gew.% Linolsäure und/oder eine derartige Menge an Linolsäurederivaten, die 0,001-10 Gew.% Linolsäure entsprechen, bevorzugt 0,01-5 Gew.% Linolsäure und/oder eine derartige Menge an Linolsäurederivaten, die 0,01-5 Gew.% Linolsäure entsprechen, jeweils bezogen auf das Gewicht der Gesamtzusammensetzung. Bevorzugt enthalten die erfindungsgemäßen Zusammensetzungen neben dem *Echinacea*-Extrakt keinen weiteren Extrakt, insbesondere keinen weiteren Pflanzenextrakt. Die Linolsäure-Quelle (die beanspruchten natürlichen Öle und Fette wie Distelöl) soll dabei erfindungsgemäß nicht als Extrakt verstanden werden.

Die erfindungsgemäßen Zusammensetzungen enthalten ferner bevorzugt einen oder mehrere kosmetische oder pharmazeutische Hilfsstoffe. Bevorzugt liegen die erfindungsgemäßen Zusammensetzungen in Form einer Emulsion und dabei besonders bevorzugt als topische Formulierung vor. Besonders geeignet ist dabei eine Creme oder eine Hautmilch. Die erfindungsgemäß verwendbaren Hilfsstoffe sind daher die in diesen technischen Gebieten üblichen Hilfsstoffe, insbesondere die bei topischen Formulierungen bekannterweise eingesetzten Materialien. Geeignete Hilfsstoffe sind beispielsweise beschrieben in der WO 2001/066076 A und der DE 10 2005 029 387 A1. Zwei besonders bevorzugte Zusammensetzungen in Form einer Creme und einer Hautmilch sind im unten stehenden Beispielteil der vorliegenden Anmeldung angeführt.

### Kurzbeschreibung der Abbildungen

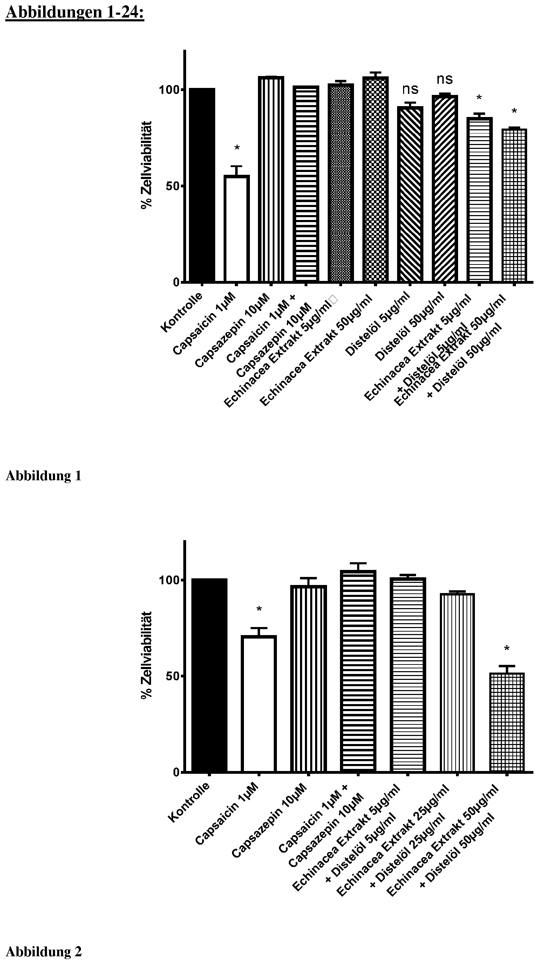

Abbildung 1 ist ein Diagramm, das die Effekte von *Echinacea*-Extrakt, Distelöl und einer Mischung von *Echinacea*-Extrakt und Distelöl im Vergleich zu den Kontrollsubstanzen Capsaicin und Capsazepin auf die Aktivierung von TRPV1 zeigt.
Abbildung 2 ist ein Diagramm, das die Dosiswirkungskurve der Effekte einer Mischung von *Echinacea*-Extrakt und Distelöl im Vergleich zu den Kontrollsubstanzen Capsaicin und Capsazepin auf die Aktivierung von TRPV1 zeigt.

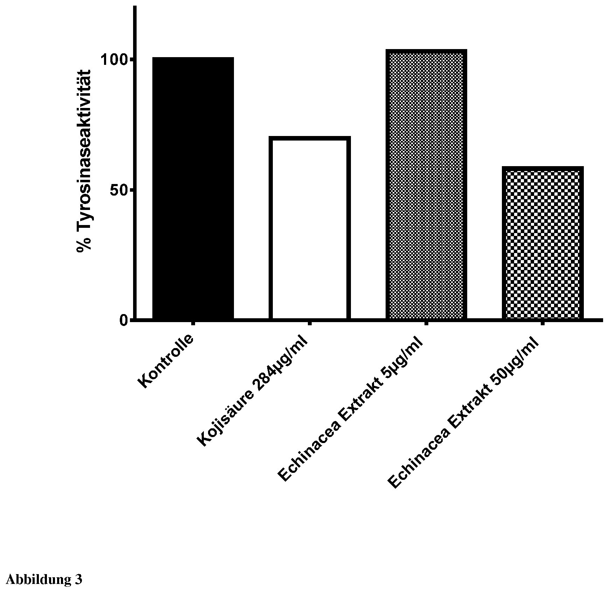

Abbildung 3 ist ein Diagramm, das den Effekt des *Echinacea*-Extrakts auf die Tyrosinase-Aktivität in B16 Melanomzellen zeigt. Als Positivkontrolle für die Hemmung der Tyrosinase-Aktivität wurde Kojisäure eingesetzt.

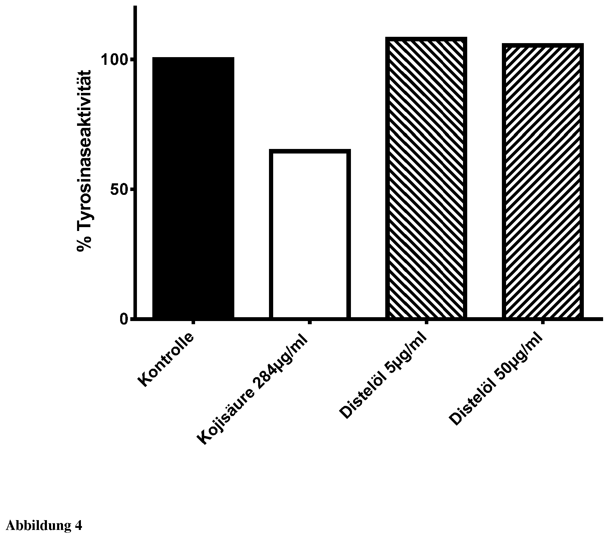

Abbildung 4 ist ein Diagramm, das den Effekt von Distelöl auf die Tyrosinase-Aktivität in B16 Melanomzellen zeigt.

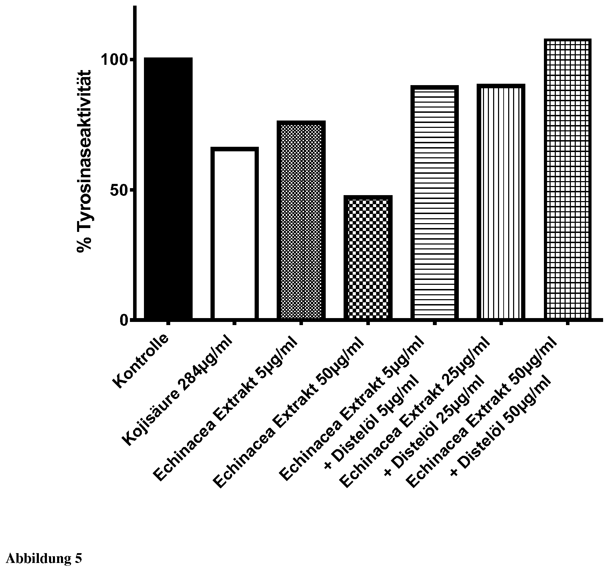

Abbildung 5 ist ein Diagramm, das den Effekt einer Mischung von *Echinacea-Extrakt* und Distelöl auf die Tyrosinase-Aktivität in B16 Melanomzellen zeigt.

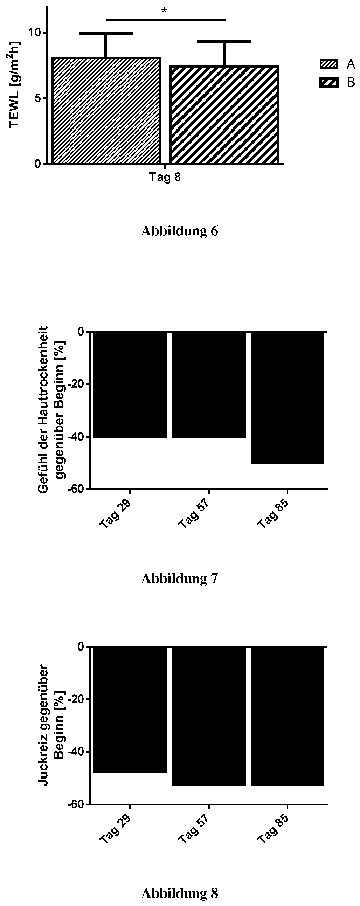

Abbildung 6 ist ein Diagramm, das den Effekt einer erfindungsgemäßen Creme mit *Echinacea*-Extrakt und Linolsäurederivaten im Vergleich zur Placebo Creme auf den transepidermalen Wasserverlust zeigt (A: Placebo Creme; B: Creme mit *Echinacea* und Linolsäurederivaten).
Abbildung 7 ist ein Diagramm, das die Reduktion des Gefühls der Hauttrockenheit nach Behandlung mit einer erfindungsgemäßen Creme mit *Echinacea-Extrakt* und Linolsäurederivaten zeigt.
Abbildung 8 ist ein Diagramm, das die Reduktion des Juckreizes nach Behandlung mit einer erfindungsgemäßen Creme mit *Echinacea-Extrakt* und Linolsäurederivaten zeigt.

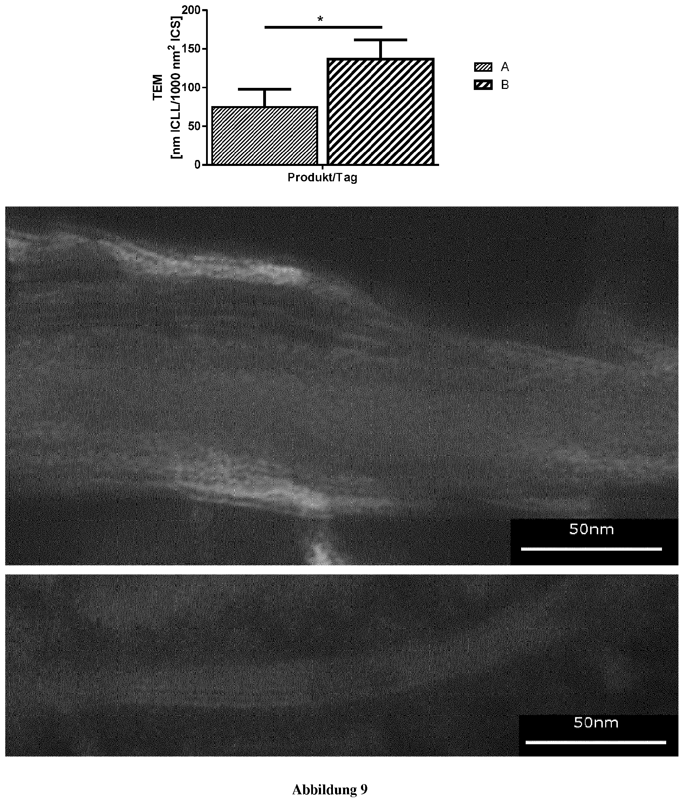

Abbildung 9 ist ein Diagramm, das den Vorher/Nachher-Vergleich der Lipidlamellen (LBV-TEM) nach Behandlung mit einer erfindungsgemäßen Creme mit *Echinacea*-Extrakt und Linolsäurederivaten zeigt (Graphik mit quantitativer Auswertung oben, A: Creme mit *Echinacea* und Linolsäurederivaten/Tag 1; B: Creme mit *Echinacea* und Linolsäurederivaten/Tag 15, repräsentative Aufnahme Transmissionselektronenmikroskop (TEM) Tag 1 Mitte, TEM Aufnahme Tag 15 unten).

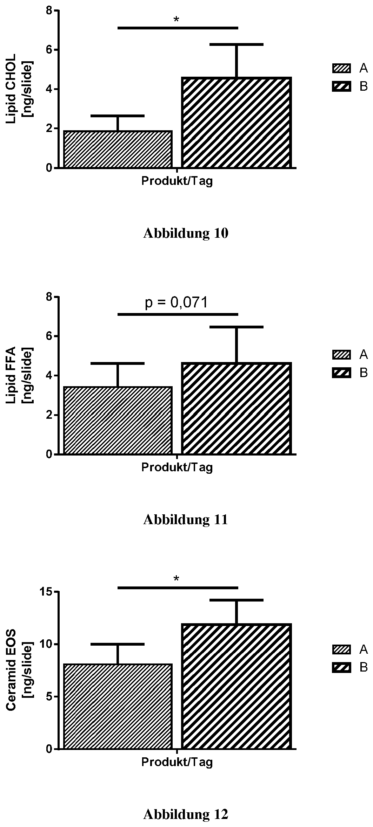

Abbildung 10 ist ein Diagramm, das den Vorher/Nachher-Vergleich des Cholesterolgehalts nach Behandlung mit einer erfindungsgemäßen Creme mit *Echinacea*-Extrakt und Linolsäurederivaten zeigt (A: Creme mit *Echinacea* und Linolsäurederivaten/Tag 1; B: Creme mit *Echinacea* und Linolsäurederivaten/Tag 15).
Abbildung 11 ist ein Diagramm, das den Vorher/Nachher-Vergleich des Gehalts an freien Fettsäuren (FFA) nach Behandlung mit einer erfindungsgemäßen Creme mit *Echinacea-*Extrakt und Linolsäurederivaten zeigt (A: Creme mit *Echinacea* und Linolsäurederivaten/Tag 1; B: Creme mit *Echinacea* und Linolsäurederivaten/Tag 15).
Abbildung 12 ist ein Diagramm, das den Vorher/Nachher-Vergleich des Gehalts an Ceramid EOS nach Behandlung mit einer erfindungsgemäßen Creme mit *Echinacea*-Extrakt und Linolsäurederivaten zeigt (A: Creme mit *Echinacea* und Linolsäurederivaten/Tag 1; B: Creme mit *Echinacea* und Linolsäurederivaten/Tag 15).

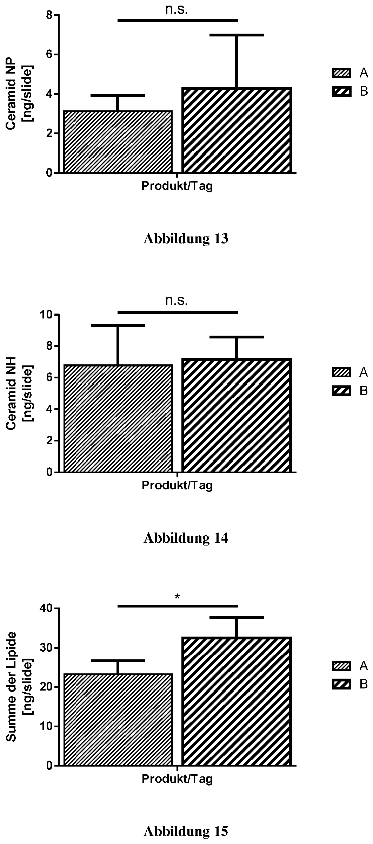

Abbildung 13 ist ein Diagramm, das den Vorher/Nachher-Vergleich des Gehalts an Ceramid NP nach Behandlung mit einer erfindungsgemäßen Creme mit *Echinacea*-Extrakt und Linolsäurederivaten zeigt (A: Creme mit *Echinacea* und Linolsäurederivaten/Tag 1; B: Creme mit *Echinacea* und Linolsäurederivaten/Tag 15).
Abbildung 14 ist ein Diagramm, das den Vorher/Nachher-Vergleich des Gehalts an Ceramid NH nach Behandlung mit einer erfindungsgemäßen Creme mit *Echinacea-Extrakt* und Linolsäurederivaten zeigt (A: Creme mit *Echinacea* und Linolsäurederivaten/Tag 1; B: Creme mit *Echinacea* und Linolsäurederivaten/Tag 15).
Abbildung 15 ist ein Diagramm, das den Vorher/Nachher-Vergleich des Gesamtgehalts an Lipiden nach Behandlung mit einer erfindungsgemäßen Creme mit *Echinacea*-Extrakt und Linolsäurederivaten zeigt (A: Creme mit *Echinacea* und Linolsäurederivaten/Tag 1; B: Creme mit *Echinacea* und Linolsäurederivaten/Tag 15).

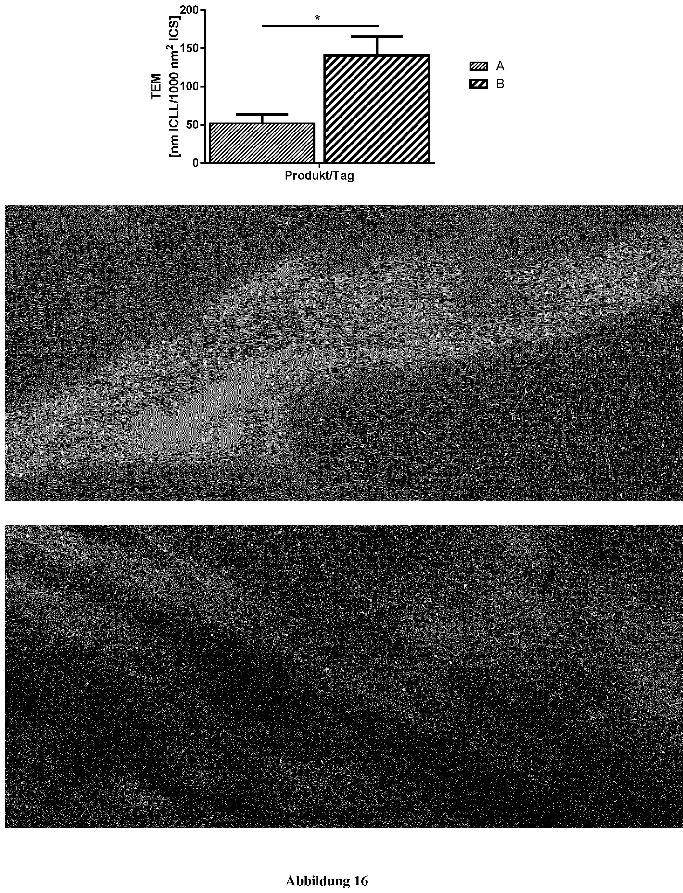

Abbildung 16 ist ein Diagramm, das den Vorher/Nachher-Vergleich der Lipidlamellen (LBV-TEM) nach Behandlung mit einer erfindungsgemäßen Hautmilch mit *Echinacea-Extrakt* und Linolsäurederivaten zeigt (Graphik mit quantitativer Auswertung oben, A: Hautmilch mit *Echinacea* und Linolsäurederivaten/Tag 1; B: Hautmilch mit *Echinacea* und Linolsäurederivaten/Tag 15, repräsentative Aufnahme Transmissionselektronenmikroskop (TEM) Tag 1 Mitte, TEM Aufnahme Tag 15 unten).

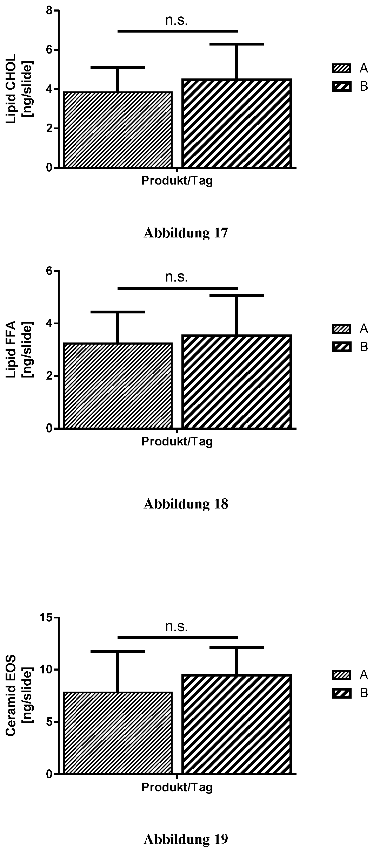

Abbildung 17 ist ein Diagramm, das den Vorher/Nachher-Vergleich des Cholesterolgehalts nach Behandlung mit einer erfindungsgemäßen Hautmilch mit *Echinacea-Extrakt* und Linolsäurederivaten zeigt (A: Hautmilch mit *Echinacea* und Linolsäurederivaten/Tag 1; B: Hautmilch mit *Echinacea* und Linolsäurederivaten/Tag 15).
Abbildung 18 ist ein Diagramm, das den Vorher/Nachher-Vergleich des Gehalts an freien Fettsäuren (FFA) nach Behandlung mit einer erfindungsgemäßen Hautmilch mit *Echinacea-*Extrakt und Linolsäurederivaten zeigt (A: Hautmilch mit *Echinacea* und Linolsäurederivaten/Tag 1; B: Hautmilch mit *Echinacea* und Linolsäurederivaten/Tag 15).
Abbildung 19 ist ein Diagramm, das den Vorher/Nachher-Vergleich des Gehalts an Ceramid EOS nach Behandlung mit einer erfindungsgemäßen Hautmilch mit *Echinacea-Extrakt* und Linolsäurederivaten zeigt (A: Hautmilch mit *Echinacea* und Linolsäurederivaten/Tag 1; B: Hautmilch mit *Echinacea* und Linolsäurederivaten/Tag 15).

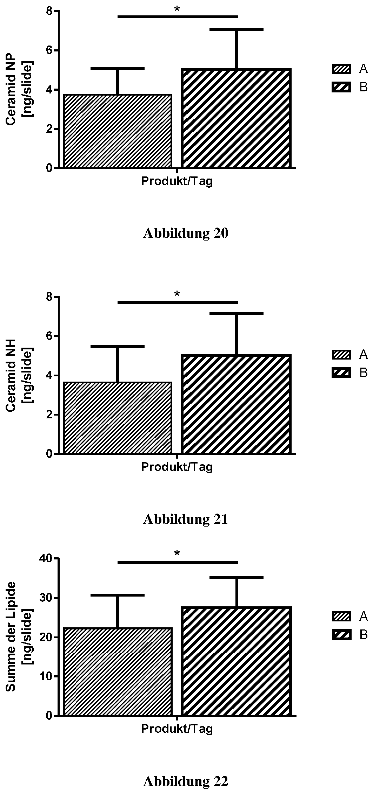

Abbildung 20 ist ein Diagramm, das den Vorher/Nachher-Vergleich des Gehalts an Ceramid NP nach Behandlung mit einer erfindungsgemäßen Hautmilch mit *Echinacea*-Extrakt und Linolsäurederivaten zeigt (A: Hautmilch mit *Echinacea* und Linolsäurederivaten/Tag 1; B: Hautmilch mit *Echinacea* und Linolsäurederivaten/Tag 15).
Abbildung 21 ist ein Diagramm, das den Vorher/Nachher-Vergleich des Gehalts an Ceramid NH nach Behandlung mit einer erfindungsgemäßen Hautmilch mit *Echinacea-Extrakt* und Linolsäurederivaten zeigt (A: Hautmilch mit *Echinacea* und Linolsäurederivaten/Tag 1; B: Hautmilch mit *Echinacea* und Linolsäurederivaten/Tag 15).
Abbildung 22 ist ein Diagramm, das den Vorher/Nachher-Vergleich des Gesamtgehalts an Lipiden nach Behandlung mit einer erfindungsgemäßen Hautmilch mit *Echinacea*-Extrakt und Linolsäurederivaten zeigt (A: Hautmilch mit *Echinacea* und Linolsäurederivaten/Tag 1; B: Hautmilch mit *Echinacea* und Linolsäurederivaten/Tag 15).

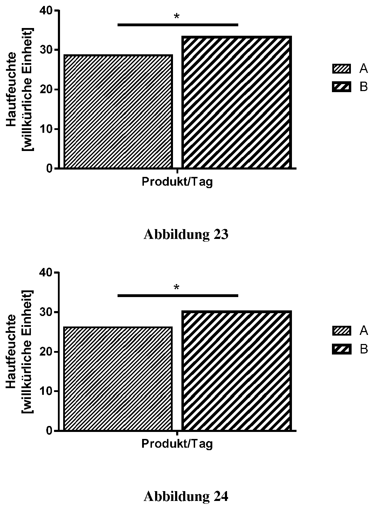

Abbildung 23 ist ein Diagramm, das den Vorher/Nachher-Vergleich der Hautfeuchte nach Behandlung mit einer erfindungsgemäßen Creme mit *Echinacea*-Extrakt und Linolsäurederivaten zeigt (A: Creme mit *Echinacea* und Linolsäurederivaten/Tag 1; B: Creme mit *Echinacea* und Linolsäurederivaten/Tag 15).
Abbildung 24 ist ein Diagramm, das den Vorher/Nachher-Vergleich der Hautfeuchte nach Behandlung mit einer erfindungsgemäßen Hautmilch mit *Echinacea-Extrakt* und Linolsäurederivaten zeigt (A: Hautmilch mit *Echinacea* und Linolsäurederivaten/Tag 1; B: Hautmilch mit *Echinacea* und Linolsäurederivaten/Tag 15).

### Beispiele

### Beispiel 1:

### Bestimmung der TRPV1 agonistischen Aktivität.

### A) Experimentelle Methode

2x10⁴ 293T-VR1 Zellen (oder elterliche 293T Zellen als Kontrolle) werden in 96-well Mikrotiterplatten (Costar, Cambridge, MA) in 200 µL Dulbecco's Modified Eagle's Medium (DMEM), welches zusätzlich 10% fötales Kälberserum und 1% Antibiotika (Penicillin/Streptomycin) enthält, kultiviert. Die Zellen werden mit den Testsubstanzen oder den Kontrollen (Capsaicin, TRPV1 Agonist (1 µM); Capsazepin, TRPV1 Antagonist (10 µM); bzw. Capsaicin (1 µM) und Capsazepin (10 µM)) für 6 h inkubiert. Anschließend wird die Zellviabilität bestimmt.

Der zytotoxische Effekt der Testsubstanzen wurde mit der MTT (3-[4,5-Dimethylthiazol-2-yl]-2,5-diphenyltetrazoliumbromid) Methode bestimmt. Dazu wurden 50 µL einer Lösung von MTT in DMEM (5 mg/mL) in jedes Well gegeben. Anschließend wurde für 4 h bei 37°C im Dunklen inkubiert. Die Reaktion wurde gestoppt, der Überstand entfernt und 100 µL pro Well DMSO wurden zugegeben. Es wurde 10 min unter schonendem Schütteln inkubiert. Anschließend wurde die Absorption bei 550 nm mit einem TECAN GENios Pro Plate Reader (Tecan Group Ltd., Schweiz) gemessen. Die Resultate wurden als Prozent der Kontrolle (Vehikellösung, 100%) dargestellt. Die Bestimmung erfolgte im Triplikat.

### B) Effekte der Testsubstanzen auf die Aktivierung von TRPV1

Abbildung 1 zeigt die Effekte von *Echinacea*-Extrakt, Distelöl und einer Mischung von *Echinacea*-Extrakt und Distelöl im Vergleich zu den Kontrollsubstanzen Capsaicin und Capsazepin auf die Aktivierung von TRPV1.
Durch die Behandlung mit der Positivkontrolle, dem TRPV1 Agonisten Capsaicin wurde die Zellviabilität auf 54,9% reduziert. Der TRPV1 Antagonist Capsazepin allein zeigte keine Wirkung (106,1% Zellviabilität). Der Effekt von Capsaicin konnte mit Capsazepin blockiert werden (101,3% Zellviabilität), was die Vermittlung des zytotoxischen Effekts über TRPV1 beweist. Bei den mit dem Extrakt aus *Echinacea* (5 µg/mL oder 50 µg/mL) behandelten Zellen wurden keine zytotoxischen Effekte beobachtet (102,3 bzw. 106,0% Zellviabilität). Bei den mit Distelöl (5 µg/mL oder 50 µg/mL) behandelten Zellen wurde lediglich ein kleiner, nicht signifikanter, zytotoxischer Effekt beobachtet (90,5 bzw. 96,5%). Erstaunlicherweise zeigte es sich, dass die mit einer Mischung des Extrakts aus *Echinacea* und Distelöl (jeweils 5 µg/mL oder jeweils 50 µg/mL) einen deutlichen, signifikanten, zytotoxischen Effekt. Mit 85,0% Zellviabilität war die Effektgröße selbst für die niedrigere Konzentration größer als für Distelöl allein. Für die höhere Konzentration wurde sogar eine Zellviabilität von lediglich 79,3% bestimmt.

Überraschenderweise zeigten diese Ergebnisse, dass es einen synergistische Effekt der Mischung aus dem Extrakt aus *Echinacea* und Distelöl auf die Aktivierung von TRPV1 gibt, der bereits bei niedrigeren Konzentrationen auftritt und die Effekte der Einzelsubstanzen deutlich übersteigt.

Abbildung 2 zeigt die Dosiswirkungskurve der Effekte einer Mischung von *Echinacea-*Extrakt und Distelöl im Vergleich zu den Kontrollsubstanzen Capsaicin und Capsazepin auf die Aktivierung von TRPV1.

In diesem Experiment fiel die agonistische Aktivität der Positivkontrolle Capsaicin etwas geringer aus (70,6% Zellviabilität). Wie im vorangegangenen Experiment konnte der Effekt von Capsaicin wieder mit dem TRPV1 Antagonisten Capsazepin inhibiert werden (104,4% Zellviabilität). Die Mischung von *Echinacea*-Extrakt und Distelöl zeigte dosisabhängige agonistische Effekte. Für die höchste Konzentration (jeweils 50 µg/mL) wurde eine Effektgröße (51,2% Zellviabilität) erzielt, die den Effekt von Capsaicin übertrifft.

### Beispiel 2:

### Inhibition der Tyrosinase-Aktivität in B16 Melanomzellen

### A) Experimentelle Methode

Für die Bestimmung der Tyrosinase-Aktivität wurden Maus B16 Melanomzellen (4A5, ATCC) in Dulbecco's Modified Eagle's Medium (DMEM), welches zusätzlich 10% fötales Kälberserum und 1% Antibiotika (Penicillin/Streptomycin) enthielt, kultiviert. Die Zellen wurden drei Tage mit verschiedenen Konzentrationen der Testsubstanzen behandelt. Anschließend wurden die Zellen bei 4°C für 30 min mit einem Lysepuffer (20 mM Natriumphosphat, pH 6,8, 1% Triton X-100, 1 mM PMSF, 1 mM EDTA), der einen Proteaseinhibitorcocktail enthält, lysiert. Die Lysate wurden mit 15.000x g für 10 min zentrifugiert. Der Überstand wurde als Quelle der Tyrosinase eingesetzt. Die Reaktionsmischung zur Bestimmung der Tyrosinase-Aktivität enthält 20 mM Phosphatpuffer, pH 6,8, und 1,25 µM L-Dopa (Sigma-Aldrich). Nach Inkubation für 30 min bei 37°C wurde die Bildung von Dopachrom bestimmt. Dazu wurde die Absorption bei einer Wellenlänge von 475 nm in einem Mikrotiterplatten-Reader (TriStar LB 941, Berthold Technologies, GmbH & Co. KG) gemessen. Als Positivkontrolle für die Inhibition der Tyrosinase-Aktivität wurde Kojisäure eingesetzt.

### B) Inhibition der Tyrosinase-Aktivität durch die Testsubstanzen

Abbildung 3 zeigt die Inhibition der Tyrosinase-Aktivität durch den Extrakt aus *Echinacea* im Vergleich zu Kojisäure.

Für die Positivkontrolle Kojisäure wurde eine Inhibition der Tyrosinase-Aktivität von 30,2% bestimmt. Der *Echinacea*-Extrakt zeigte bei einer Testkonzentration von 5 µg/mL keine Inhibition der Tyrosinase-Aktivität. Für die höhere Testkonzentration von 50 µg/mL wurde eine Inhibition von 41,8% bestimmt. Somit war der Effekt größer als der der Positivkontrolle. Abbildung 4 zeigt die Inhibition der Tyrosinase-Aktivität durch Distelöl im Vergleich zu Kojisäure.

Kojisäure als Positivkontrolle zeigte eine Inhibition der Tyrosinase-Aktivität von 35,3%. Für Distelöl konnte bei keiner der beiden Testkonzentrationen eine Inhibition der Tyrosinase-Aktivität beobachtet werden.

Abbildung 5 zeigt die Inhibition der Tyrosinase-Aktivität durch den Extrakt aus *Echinacea* und einer Mischung von *Echinacea*-Extrakt und Distelöl im Vergleich zu Kojisäure. Wie in den beiden vorangegangenen Experimenten wurde für die Positivkontrolle Kojisäure wieder eine Inhibition der Tyrosinase-Aktiviät beobachtet. Diese lag bei 34,3%. Für den Extrakt aus *Echinacea* wurden für beide Konzentrationen (5 µg/mL und 50 µg/mL) eine Inhibition der Tyrosinase-Aktivität von 24,3 bzw. 52,8% gefunden. Dies bestätigte die Resultate des ersten Experiments. Überraschenderweise wurde für die Mischungen von *Echinacea-Extrakt* und Distelöl keine Inhibition der Tyrosinase-Aktivität gefunden. Diese lag bei 89,4-107,5% der Kontrolle.

Diese Ergebnisse zeigen, dass durch die Zugabe von Distelöl zum Extrakt aus *Echinacea* die Inhibition der Tyrosinase-Aktivität bei gleichbleibender Testkonzentration des *Echinacea-*Extrakts aufgehoben werden kann.

### Beispiel 3:

### Regeneration der Hautbarriere nach Behandlung mit Natriumdodecylsulfat (SDS).

Zweck der Studie war die Bestimmung des Einflusses einer erfindungsgemäßen Zusammensetzung auf die Regeneration der gestörten Barrierefunktion nach einer vierundzwanzigstündigen Behandlung mit Natriumdodecylsulfat (Patch Test).

### A) Studiendesign

Bei der Studie handelte es sich um eine randomisierte, doppelblinde Studie mit intra-individuellem Vergleich an hautgesunden Probanden (n =23).

### B) Durchführung der Studie

An Tag 1 der Studie wurde eine Lösung mit 0,5% SDS okklusiv unter Verwendung von Patches (Finn Chambers®, extra large, auf Scanpore®) für 24 Stunden auf den volaren Unterarm angewendet. Nach Entfernung des Patches an Tag 2 wurde der Barriereschaden gemessen. An den Tagen 2 bis 11 wurden die Testareale zweimal täglich mit den Testprodukten behandelt. Instrumentelle Messungen wurden an den Tagen 3, 5 und 8 durchgeführt.

Als Testprodukte wurden eine erfindungsgemäße Creme mit *Echinacea-Extrakt* (0,5 Gew.%) und Linolsäurederivaten (entsprechend der unten angeführten Beispielcreme) und eine Placebo Creme eingesetzt. Die Applikation der Testprodukte erfolgte zweimal täglich auf einer Fläche von jeweils 5 cm x 6 cm. Dabei wurden 2 mg/cm² der Testsubstanzen aufgetragen.

Die Probanden waren männlich oder weiblich. Ihr Alter lag zwischen 18 und 55 Jahren.

### Bestimmung des transepidermalen Wasserverlusts:

Der transepidermale Wasserverlust (TEWL) ist eine nicht-invasive Methode zur Bestimmung der Integrität der Barrierefunktion des Stratum corneums und wird als sensitiver Parameter für die Tensid-induzierte Hautirritation angesehen. Der TEWL wurde mit einem DermaLab (Cortex, Dänemark) bestimmt (Dreifachbestimmung). Die Messsonde wurde für jede Messung für 40 s auf dem zu vermessenden Hautareal platziert. Die ersten 20 s dienten der Äquilibrierung des Systems. Die Werte der zweiten 20 s wurden gemittelt und als Messwert gewertet. Werte für TEWL wurden in der Einheit g/m²h angegeben. Der übliche Bereich für normale, nicht-irritierte Haut liegt zwischen 3 und 9 g/m²h. Leicht irritierte Haut mit einer gestörten Hautbarriere zeigt üblicherweise abhängig vom Ausgangswert der Probanden Werte zwischen 10 und 18 g/m²h. Stark irritierte Haut mit einer deutlich gestörten Hautbarriere ruft Werte von >25 g/m²h hervor. Ein Anstieg der TEWL-Werte ist somit indikativ für eine Störung der Funktion der Hautbarriere.

### C) Effekte der Testprodukte auf die Hautbarriere

Abbildung 6 zeigt den Effekt der erfindungsgemäßen Creme mit *Echinacea-Extrakt* und Linolsäurederivaten im Vergleich zur Placebo Creme auf den transepidermalen Wasserverlust.

Die Applikation beider Testprodukte führte zu einer deutlichen Reduktion des transepidermalen Wasserverlusts, der an Tag 2 >20 g/m²h betrug. Überraschenderweise zeigten die Ergebnisse, dass der Effekt der erfindungsgemäßen Creme mit *Echinacea*-Extrakt und Linolsäurederivaten auf die Reduktion des transepidermalen Wasserverlust an Tag 8 signifikant größer ist als der der Placebo Creme.

### Beispiel 4:

### Studie in Patienten mit atopischer Dermatitis (3 Monate)

### A) Studiendesign

Bei der Studie handelte es sich um eine doppelblinde, randomisierte, Vergleichsprodukt-kontrollierte Studie mit intra-individuellem Vergleich.

### B) Durchführung der Studie

Als Prüfpräparate wurden die erfindungsgemäße Creme mit *Echinacea-Extrakt* (0,5 Gew.%) und Linolsäurederivaten (entsprechend der unten angeführten Beispielcreme) und als Vergleichspräparat eine herkömmliche Creme (W/Ö-Creme, Fettgehalt ca. 55 %, ca. 2,5 % Pflanzen-Extrakt enthaltend Betulin) eingesetzt. Die Produkte wurden zweimal täglich angewendet. Die Applikationsfläche betrug je 1600 cm² (40 x 40 cm, ca. 9 % der Oberfläche eines erwachsenen Mannes) für jedes Produkt auf kontralateralen Hautstellen. Als Applikationsstellen wurden die Ellenbogenbeugen und der ganzen Arm oder alternativ die Kniekehlen und das halbe Bein oberhalb und unterhalb des Knies oder die halbe Oberkörperseite ausgewählt. Die Produkte wurden für ca. 3 Monate (85 Tage) angewendet. Die Applikationsmenge pro Produktanwendung betrug 1,5 g. An den Tagen 1 (Beginn), 29 (1 Monat), 57 (2 Monate) und 85 (3 Monate) erfolgte die Beurteilung des Juckreizes durch den Prüfarzt sowie die subjektive Beurteilung des Trockenheitsgefühls durch den Patienten.

### C) Abnahme des Gefühls der Hauttrockenheit und des Juckreizes nach Behandlung mit der erfindungsgemäßen Creme mit Echinacea-Extrakt und Linolsäurederivaten (n =46-48)

Abbildung 7 zeigt den zeitlichen Verlauf Reduktion des Gefühls der Hauttrockenheit nach Behandlung mit der erfindungsgemäßen Creme mit *Echinacea*-Extrakt und Linolsäurederivaten.

Das Gefühl der Hauttrockenheit nahm nach Behandlung mit der erfindungsgemäßen Creme mit *Echinacea*-Extrakt und Linolsäurederivaten an Tag 29 um 40% gegenüber dem Behandlungsbeginn ab. An den Tagen 57 und 85 betrug die Reduktion 40 bzw. 50%. Die Abnahme des Gefühls der Hauttrockenheit an Tag 85 war signifikant größer als des Vergleichspräparats (-35%).

Abbildung 8 zeigt den zeitlichen Verlauf Reduktion des Juckreizes nach Behandlung mit der erfindungsgemäßen Creme mit *Echinacea-Extrakt* und Linolsäurederivaten.

Der Juckreiz nahm nach Behandlung mit der erfindungsgemäßen Creme mit *Echinacea-*Extrakt und Linolsäurederivaten an Tag 29 um 47,6% gegenüber dem Behandlungsbeginn ab. An den Tagen 57 und 85 betrug die Reduktion des Pruritus 52,4%. Die Abnahme des Juckreizes an den Tagen 57 und 85 war signifikant größer als die des Vergleichspräparats (jeweils -33,3%).

### Beispiel 5:

### 14 d Studie in Patienten mit atopischer Dermatitis

### A) Studiendesign

Die Studie wurde mit einem intra-individuellem, doppelt verblindeten Vergleich durchgeführt.

### B) Durchführung der Studie

Die Studie wurde mit Probanden mit atopischer Diathese (Alter von 18 bis 70 Jahren (22 weiblich, 3 männlich, lokaler SCORAD zwischen 1 und 15)) durchgeführt. Als Prüfpräparate wurden die erfindungsgemäße Creme mit *Echinacea*-Extrakt (0,5 Gew.%) und Linolsäurederivaten und die erfindungsgemäße Hautmilch mit *Echinacea*-Extrakt (0,1 Gew.%) und Linolsäurederivaten (entsprechend der unten angeführten Beispielcreme und Beispielhautmilch) eingesetzt. Die Produkte wurden für 14 Tage zweimal täglich angewendet.

Die Hautfeuchte an Tag 1 und an Tag 15 wurde mittels Corneometrie bestimmt.

Die Untersuchung der epidermale Hautbarriere direkt ohne invasive Probennahmen erfolgte mit dem Analyseverfahren Lipbarvis® (Lipid Barrier Visualisation, LBV) durch die Firma Microscopy Services Dähnhardt (Flintbek, Deutschland). Dafür wurden Hautproben im Umfang von drei bis fünf Zelllagen durch ein besonders schonendes Kleber/Träger-System entnommen.

### Vorher/Nachher-Vergleiche der Lipidlamellen (LBV-TEM):

Die Proben wurden präpariert und im Transmissionselektronenmikroskop untersucht. Eine spezielle Software ermittelte die Länge der Lipidlamellen und setzte diese in Relation zum Interzellularraum. Die Anzahl der Lipidlamellen lässt sehr genaue Rückschlüsse auf die Wirksamkeit des aufgetragenen Wirkstoffs zu.

### Bestimmung der Hautlipide (LBV-LIP):

Durch die HPTLC-Analyse (High Performance Thin Layer Chromatography) der Hautprobe auf Gesamt- und Einzellipide wurden die wichtigen Hautlipide wie Cholesterol, Fettsäuren, Ceramide EOS, NP und NH quantitativ bestimmt.

### C) Zunahme der Lipidlamellen nach Behandlung mit der erfindungsgemäßen Creme mit Echinacea-Extrakt und Linolsäurederivaten oder der erfindungsgemäßen Hautmilch mit Echinacea-Extrakt und Linolsäurederivaten, Erhöhung der Synthese hauteigener Lipide und Erhöhung der Hautfeuchte (n = 20)

Abbildungen 9 (Creme) und 16 (Hautmilch) zeigen den Vorher/Nachher Vergleich der Lipidlamellen (LBV-TEM) nach Behandlung mit einem Produkt *Echinacea*-Extrakt und Linolsäurederivate enthaltend.

In beiden Fällen wurde erfindungsgemäß ein signifikanter Anstieg der Lipidlamellenzahl gefunden.

Abbildungen 10 (Creme) und 17 (Hautmilch) zeigen den Vorher/Nachher Vergleich des Cholesterol-Gehalts nach Behandlung mit einem Produkt *Echinacea*-Extrakt und Linolsäurederivate enthaltend.

Für beide Produkte wurde ein Anstieg des Gehalts an Cholesterol gefunden. Für die erfindungsgemäße Creme mit *Echinacea*-Extrakt und Linolsäurederivaten war der Unterschied besonders signifikant.
Abbildungen 11 (Creme) und 18 (Hautmilch) zeigen den Vorher/Nachher Vergleich des Gehalts an freien Fettsäuren (FFA) nach Behandlung mit einem Produkt *Echinacea*-Extrakt und Linolsäurederivate enthaltend.

Für beide Produkte wurde erfindungsgemäß ein Anstieg des Gehalts an freien Fettsäuren gefunden.

Abbildungen 12 (Creme) und 19 (Hautmilch) zeigen den Vorher/Nachher Vergleich des Gehalts an Ceramid EOS nach Behandlung mit einem Produkt *Echinacea*-Extrakt und Linolsäurederivate enthaltend.

Für beide Produkte wurde erfindungsgemäß ein Anstieg des Gehalts an Ceramid EOS gefunden. Für die erfindungsgemäße Creme mit *Echinacea*-Extrakt und Linolsäurederivaten war der Unterschied besonders signifikant.

Abbildungen 13 (Creme) und 20 (Hautmilch) zeigen den Vorher/Nachher Vergleich des Gehalts an Ceramid NP nach Behandlung mit einem Produkt *Echinacea*-Extrakt und Linolsäurederivate enthaltend.

Für beide Produkte wurde erfindungsgemäß ein Anstieg des Gehalts an Ceramid NP gefunden. Für die erfindungsgemäße Hautmilch mit *Echinacea*-Extrakt und Linolsäurederivaten war der Unterschied besonders signifikant.

Abbildungen 14 (Creme) und 21 (Hautmilch) zeigen den Vorher/Nachher Vergleich des Gehalts an Ceramid NH nach Behandlung mit einem Produkt *Echinacea*-Extrakt und Linolsäurederivate enthaltend.

Für beide Produkte wurde erfindungsgemäß ein Anstieg des Gehalts an Ceramid NH gefunden. Für die erfindungsgemäße Hautmilch mit *Echinacea*-Extrakt und Linolsäurederivaten war der Unterschied besonders signifikant.

Abbildungen 15 (Creme) und 22 (Hautmilch) zeigen den Vorher/Nachher Vergleich des Gesamtgehalts an Lipiden nach Behandlung mit einem Produkt *Echinacea*-Extrakt und Linolsäurederivate enthaltend.

Für beide Produkte wurde erfindungsgemäß ein Anstieg des Gesamtgehalts an Lipiden gefunden. Der Unterschied war für beide Gruppen signifikant.

Abbildungen 23 (Creme) und 24 (Hautmilch) zeigen den Vorher/Nachher Vergleich der Hautfeuchte nach Behandlung mit einem Produkt *Echinacea*-Extrakt und Linolsäurederivate enthaltend.

Nach Behandlung mit der erfindungsgemäßen Creme mit *Echinacea*-Extrakt und Linolsäurederivaten oder der erfindungsgemäßen Hautmilch mit *Echinacea-Extrakt* und Linolsäurederivaten war die Hautfeuchte signifikant erhöht.

### Beispiel 6:

### Klinische Studie zur Untersuchung des Irritations- und Sensibilisierungspotentials

### A) Studiendesign

Bei der Studie handelte es sich um eine doppelblinde, randomisierte, monozentrische Vergleichsprodukt-kontrollierte Studie.

### B) Durchführung der Studie

Die Studie wurde mit 105 freiwilligen Probanden (Alter von 18 bis 75 Jahren, weiblich und männlich, Hautphototyp I-IV, keine akuten Hauterkrankungen an den exponierten Flächen) durchgeführt. Als Prüfpräparate wurden die erfindungsgemäße Creme mit *Echinacea*-Extrakt (0,5 Gew.%) und Linolsäurederivaten und die erfindungsgemäße Hautmilch mit *Echinacea-*Extrakt (0,1 Gew.%) und Linolsäurederivaten (entsprechend der unten angeführten Beispielcreme und Beispielhautmilch) sowie das Placebo der erfindungsgemäßen Creme mit *Echinacea*-Extrakt und Linolsäurederivaten eingesetzt.

### Irritations-/Induktionsphase:

Die Produkte wurden okklusiv mit FinnChamber® Large (18 mm) auf Fixomull® Stretch kontinuierlich auf die gleiche Patchstelle über 14 Tage aufgetragen. Es wurde sechsmal über 48 oder 72 Stunden appliziert (150 µL pro Applikation). Die Testareale befanden sich am unteren Rücken. Die Bewertung der Erytheme erfolgte visuell mit einer Skala von 0 - 6, 10 - 30 Minuten nach der Entfernung des Patches (Tage 3, 5, 8, 10, 12, 15).

### Challengephase (eine Woche Erholung nach Induktionsphase):

Die Produkte wurden okklusive mit FinnChamber® Large (18 mm) auf Fixomull® Stretch aufgetragen. Es wurde einmal über 48 Stunden appliziert (150 µL pro Applikation). Die Testareale befanden sich am oberen Arm. Die Bewertung der Erytheme erfolgte visuell mit einer Skala von 0 - 6, 10 - 30 Minuten und 48 Stunden nach der Entfernung des Patches.

### Statistische Methoden:

### Irritationspotential:

Der Cumulative Irritation Score für jedes Subjekt wurde als Summer der Scores während der Irritationsphase berechnet. Für fehlende Werte wurde der Werte der letzten Beobachtung übertragen. Wenn ein Grad von 2 oder höher aufgenommen wurde, wurde der Werte des Grades 2 übertragen. Eine deskriptive Statistik (Mittelwert, Standardabweichung, Minimum, Maximum und Median) für den Cumulative Irritation Score wurde erstellt.

### Sensibilisierungspotential:

Die folgenden Kategorien des Sensibilisierungspotentials wurden verwendet:
Niedrig: Kein Subjekt reagiert mit einem Grad 2 oder höher oder bis zu zwei Subjekte mit Grad 1 zu beiden Bewertungszeitpunkten während der Sensibilisierungsphase.
Moderat: Bis zu zwei Subjekte reagieren mit einem Grad 2 oder höher oder bis zu vier Subjekte mit Grad 1 zu beiden Bewertungszeitpunkten während der Sensibilisierungsphase. Hoch: Mehr als zwei Subjekte reagieren mit einem Grad 2 oder höher oder mehr als vier Subjekte mit Grad 1 zu beiden Bewertungszeitpunkten während der Sensibilisierungsphase.

### C) Irritations- und Sensibilisierungspotentials

Es wurde ein niedriges bis mäßiges Irritationspotential für alle getesteten Produkte gefunden. Dabei gab es keine statistisch signifikanten Unterschiede zwischen den Gruppen.
Eine 48stündige okklusive Applikation der Testprodukte während der Challengephase resultierte an den Tagen 24 und 26 nicht in der Bildung von Erythemen des Grades 1 oder höher. Gemäß Protokoll wurden alle Produkte als Produkte mit niedrigem Sensibilisierungspotential kategorisiert.

Unter Berücksichtigung der mehrfachen okklusiven Applikation über 48 Stunden ohne Unterbrechung zwischen den Applikationen, die Abwesenheit von oder die niedrigen Level der Irritationen nach der ersten Applikation und die niedrigen Level von zusätzlichen klinischen Zeichen von Irritation wurde die Hautverträglichkeit aller Produkte als sehr gut bis gut kategorisiert.
Das Sensibilisierungspotential wurde als niedrig bei nicht ablesbarem allergenem Potential eingestuft.

### Beispiel 7:

### Klinische SDS-Irritationsstudie

### A) Studiendesign

Bei der Studie handelte es sich um eine verblindete Studie.

### B) Durchführung der Studie

Die Studie wurde mit 44 weiblichen Probanden durchgeführt. Alle Probanden nahmen an der 24stündigen okklusiven Behandlung mit SDS (Natriumdodecylsulfat) teil. Neun Probanden wurden nach Bewertung der Baseline wegen zu geringer oder inhomogener SDS-Irritation ausgeschlossen. Drei weitere Probanden wurden wegen einer schweren SDS-Irritation vor der Bewertung der Baseline ausgeschlossen. Die 31 Probanden, die die Studie komplett abschlossen, waren zwischen 24,9 und 65,6 Jahren alt. Als Prüfpräparate wurden die erfindungsgemäßen Cremes mit *Echinacea*-Extrakt (1 bzw. 2 Gew.%) und Linolsäurederivaten eingesetzt. Verglichen wurde mit unbehandelter Haut und mit mit SDS irritierter Haut (nicht irritiert/unbehandelt, Irritation mit SDS/unbehandelt). Primäres Ziel war die Evaluation der kosmetischen Eigenschaften der beiden erfindungsgemäßen Cremes mit *Echinacea*-Extrakt und Linolsäurederivaten. Sekundäres Ziel war die Bestimmung des Einflusses der erfindungsgemäßen Cremes mit *Echinacea-Extrakt* und Linolsäurederivaten auf die Reduktion des Erythems.

Die Hautbarriere der Probanden wurde durch eine 24stündige okklusive Applikation mit 2% SDS Lösung in FinnChamber® (18 mm) auf den Testarealen am Unterarm geschädigt. Der Transepidermale Wasserverlust (TEWL, DermaLab®) und die Hydratation der Haut (Corneometer® MDD4) wurde 4 h, 48 h, 3 d und 6 d nach der Irritation mit SDS gemessen. Zusätzlich wurde das Erythem visuell bestimmt (Einstufung durch Experten). Die beiden erfindungsgemäßen Cremes mit *Echinacea-Extrakt* und Linolsäurederivaten wurden randomisiert auf zwei Testareale aufgetragen. Ein weiteres Testareal auf dem Unterarm blieb unbehandelt. Als Kontrollfeld diente ein Testareal auf dem Oberarm (nicht irritiert/unbehandelt). Die Applikation der beiden erfindungsgemäßen Cremes mit *Echinacea-*Extrakt und Linolsäurederivaten (∼2 µL/cm²) erfolgte zweimal täglich.

Die Messungen und visuellen Bewertungen wurden zu folgenden Zeitpunkten durchgeführt:
- vor der Behandlung mit Produkt, 4 h nach dem Entfernen des Patches (t₀)
- nach der 4. Produktapplikation, 48 h nach dem Entfernen des Patches (t₁)
- nach der 6. Produktapplikation, 3 d nach dem Entfernen des Patches (t₂)
- nach der 12. Produktapplikation, 5 d nach dem Entfernen des Patches (t₃)

### C) Transepidermaler Wasserverlust (TEWL)

Die Behandlung mit der der erfindungsgemäßen Creme mit *Echinacea*-Extrakt (1 Gew.%) und Linolsäurederivaten führte zu einer im Vergleich zum unbehandelten Areal leichten, nicht signifikanten Reduktion des TEWL zu den Zeitpunkten to und t₁ An den Zeitpunkten t₂ und t₃ wurden keine großen Unterschiede zwischen den beiden Gruppen festgestellt. Nach Behandlung mit der erfindungsgemäßen Creme mit *Echinacea*-Extrakt (2 Gew.%) und Linolsäurederivaten waren die TEWL Werte generell höher als im Fall der unbehandelten Haut. Ein erhöhter TEWL ist mit einer niedrigeren Barrierefunktion gleichzusetzen. Die Ergebnisse sind in Tabelle 1 dargestellt.

**Tabelle 1: Transepidermaler Wasserverlust (TEWL), Originaldaten in g/(m²h)**

| Messzeitpunkt | SDS-irritiert/ unbehandelt | 1% Creme | 2% Creme |
|---|---|---|---|
| t₀ | 41,3 | 39,9 | 41,5 |
| t₁ | 30,7 | 28,6 | 32,0 |
| t₂ | 18,6 | 19,1 | 22,2 |
| t₃ | 10,9 | 11,5 | 13,1 |

### D) Hauthydratation (Corneometer®)

Die mit den erfindungsgemäßen Cremes mit *Echinacea*-Extrakt und Linolsäurederivaten behandelten Testareale wiesen im Vergleich zu den SDS-irritierten, unbehandelten Hautarealen an t₂ und t₃ statistisch signifikant erhöhte Werte für die Hauthydratation auf. An t₁ waren die Werte für die Hydratation der Haut ebenfalls höher. Die Ergebnisse sind in Tabelle 2 dargestellt.

**Tabelle 2: Hauthydratation, Originaldaten [AU]**

| Messzeitpunkt | SDS-irritiert/ unbehandelt | 1% Creme | 2% Creme |
|---|---|---|---|
| t₀ | 35,6 | 35,1 | 33,0 |
| t₁ | 19,3 | 20,9 | 20,6 |
| t₂ | 11,9 | 15,9 | 14,9 |
| t₃ | 9,6 | 16,1 | 12,8 |

### E) Visuelle Bestimmung des Erythems

Wie erwartet wurde auf den nicht irritierten, unbehandelten Testarealen kein Erythem beobachtet. Folglich waren die Erythem Scores auf allen irritierten Arealen signifikant höher. Zwischen den mit der der erfindungsgemäßen Creme mit *Echinacea*-Extrakt und Linolsäurederivaten behandelten Arealen und den mit SDS irritierten, unbehandelten Arealen wurde kein großer Unterschied beobachtet. Es zeigte sich aber, dass der Score für die mit der erfindungsgemäßen Creme mit *Echinacea*-Extrakt (1 Gew.%) und Linolsäurederivaten behandelten Areale an t₃ niedriger war als für die mit SDS irritierten, unbehandelten Areale. Im Gegensatz dazu wurde für die mit der erfindungsgemäßen Creme mit *Echinacea*-Extrakt (2 Gew.%) und Linolsäurederivaten behandelten Areale lediglich an t₀ ein niedrigerer Score gefunden. An allen anderen Messzeitpunkten war der Erythem Score größer als für die mit SDS irritierten, unbehandelten Areale (nicht signifikant). Die Ergebnisse sind in Tabelle 3 dargestellt.

**Tabelle 3: Visuelle Bewertung des Erythems, Originaldaten**

| Messzeitpunkt | nicht irritiert/ unbehandelt | SDS-irritiert/ unbehandelt | 1% Creme | 2% Creme |
|---|---|---|---|---|
| t₀ | 0,00 | 1,69 | 1,69 | 1,61 |
| t₁ | 0,00 | 1,44 | 1,47 | 1,50 |
| t₂ | 0,00 | 1,19 | 1,21 | 1,29 |
| t₃ | 0,00 | 0,94 | 0,87 | 0,97 |

Zusammenfassend wurde festgestellt, dass die erfindungsgemäßen Cremes mit *Echinacea-*Extrakt (1 bzw. 2 Gew.%) und Linolsäurederivaten einen positiven Einfluss auf die Hautfeuchtigkeit haben. Dieser Vorteil überwiegt die ebenfalls beobachtete leicht erhöhte Irritation beim Einsatz der höheren Konzentrationen des *Echinacea*-Extrakts.

### Beispiel 8:

### Stabilität der Formulierungen

Die Creme mit *Echinacea*-Extrakt (0,5 Gew.%) und Linolsäurederivaten und die Hautmilch mit *Echinacea-Extrakt* (0,1 Gew.%) und Linolsäurederivaten (entsprechend der unten angeführten Beispielcreme und Beispielhautmilch) wurden einer Langzeitstabilitätsprüfung unterzogen. Hierzu wurden Proben bei verschiedenen Bedingungen (25°C/60% RH, 30°C/65% RH und 40°C/75% RH), eingelagert. Die Ergebnisse der Untersuchungen ergaben, dass die Haltbarkeit der beiden Zusammensetzungen wenigstens zwei Jahre bei Raumtemperatur beträgt.

Überraschenderweise konnte in den oben aufgeführten Beispielen 3 bis 5 gezeigt werden, das die erfindungsgemäße Creme und die erfindungsgemäße Hautmilch mit *Echinacea*-Extrakt und Linolsäurederivaten einen positiven Einfluss auf die Barrierefunktion der Haut haben.
Die Behandlung führte zu einer signifikanten Erhöhung der hauteigenen Lipide, welche zur Wiederherstellung bzw. Aufrechterhaltung der Barriere essentiell sind. Insbesondere wurde auch der Gehalt an Ceramid EOS erhöht, welches für die Stabilität der Stratum corneum-Lipiddoppelschichten wegen der sogenannten "Nagelfunktion" besonders wichtig ist.

Makroskopisch konnte in den Studien eine Verringerung des transepidermalen Wasserverlust (TEWL) sowie eine Erhöhung der Hautfeuchte nachgewiesen werden. Weiterhin war der Juckreiz nach Behandlung mit den erfindungsgemäßen Produkten reduziert.

Die oben aufgeführten Beispiele belegen, dass überraschenderweise nicht nur wie in Yotsawimonwat S, et al., Int J Cosm Sci 2010, 32, 340-346 beschrieben, die Hydratisierung der Haut in Probanden ohne Barriereschädigung verbessert werden kann, sondern dass die erfindungsgemäßen Zusammensetzungen auch zur Regeneration einer bereits gestörten Barriere beitragen. Insbesondere ist hierbei hervorzuheben, dass sich die erfindungsgemäßen Zusammensetzungen nicht nur durch die zusätzliche Anwesenheit von Linolsäure/Linolsäurederivaten von den bekannten *Echinacea* enthaltenden Zusammensetzungen unterscheiden. Auch die eingesetzten Extrakte aus *Echinacea purupurea* unterscheiden sich. Yotsawimonwat et al. setzten einen alkoholischen Extrakt aus den oberirdischen Teilen von *Echinacea* ein, der einen hohen Anteil an phenolischen Bestandteilen mit anti-oxidativer Wirkung enthält. Der Anteil an Alkylamiden ist in solchen Extrakten sehr gering. Die erfindungsgemäßen Zusammensetzungen enthalten bevorzugt einen Extrakt aus den Wurzeln von *Echinacea.* Dieser wurde bevorzugt mittels überkritischem CO₂ gewonnen, enthält also einen hohen Anteil an den lipophilen Alkylamiden und keine Phenole.

Weiterhin zeigte es sich überraschenderweise, dass die erfindungsgemäßen Zusammensetzungen über einen Zeitraum von mehr als zwei Jahren haltbar sind. Dies ist auch bei Lagerung bei Raumtemperatur der Fall. Die von Yotsawimonwat et al. beschriebenen Formulierungen waren hingegen nach Zusatz eines Antioxidans bei einer Lagerung bei 4°C maximal 7 Monate lagerbar.

Es war weiterhin für den Fachmann überraschend, dass die erfindungsgemäße Zusammensetzung antipruritische Effekte zeigt.

### Beispiele für Zusammensetzungen/Formulierungen gemäß der vorliegenden Erfindung:

**Creme mit 2% Echinacea purpurea radix CO₂-Extrakt:**

| | |
|---|---|
| Arlacel 1690 | 1,600 kg |
| Hexyldecyllaurat | 2.000 kg |
| Bienenwachs | 0,375 kg |
| Orange Peel Wachs | 0,450 kg |
| Decyloleat | 1,200 kg |
| Oxynex LM | 0,030 kg |
| Zinkstearat | 0,375 kg |
| Distelöl | 2,550 kg |
| Isopropylmyristat | 1,500 kg |
| *Echinacea purpurea radix* CO₂-Extrakt | 0,600 kg |
| Benzylalkohol | 0,300 kg |
| Magnesiumsulfat-Heptahydrat | 0,150 kg |
| Glycerol | 0,900 kg |
| Wasser, gereinigt | 17,970 kg |

**Creme mit 1% Echinacea purpurea radix CO₂-Extrakt:**

| | |
|---|---|
| Arlacel 1690 | 1,600 kg |
| Hexyldecyllaurat | 2.000 kg |
| Bienenwachs | 0,375 kg |
| Orange Peel Wachs | 0,450 kg |
| Decyloleat | 1,200 kg |
| Oxynex LM | 0,030 kg |
| Zinkstearat | 0,375 kg |
| Distelöl | 2,550 kg |
| Isopropylmyristat | 1,500 kg |
| *Echinacea purpurea radix* CO₂-Extrakt | 0,300 kg |
| Benzylalkohol | 0,300 kg |
| Magnesiumsulfat-Heptahydrat | 0,150 kg |
| Glycerol | 0,900 kg |
| Wasser, gereinigt | 18,270 kg |

**Creme mit 0,5% Echinacea purpurea radix CO₂-Extrakt:**

| | |
|---|---|
| Arlacel 1690 | 1,600 kg |
| Hexyldecyllaurat | 2.000 kg |
| Bienenwachs | 0,375 kg |
| Orange Peel Wachs | 0,450 kg |
| Decyloleat | 1,200 kg |
| Oxynex LM | 0,030 kg |
| Zinkstearat | 0,375 kg |
| Distelöl | 2,550 kg |
| Isopropylmyristat | 1,500 kg |
| *Echinacea purpurea radix* CO₂-Extrakt | 0,150 kg |
| Benzylalkohol | 0,300 kg |
| Magnesiumsulfat-Heptahydrat | 0,150 kg |
| Glycerol | 0,900 kg |
| Wasser, gereinigt | 18,420 kg |

**Hautmilch mit 0,1 % Echinacea purpurea radix CO₂-Extrakt:**

| | |
|---|---|
| Zinkstearat | 0,300 kg |
| Orange Peel Wax | 0,200 kg |
| Arlacel 1690 | 1,500 kg |
| Hexyldecyllaurat | 2,500 kg |
| Distelöl | 2,550 kg |
| Decyloleat | 1,500 kg |
| Isopropylmyristat | 1,200 kg |
| Benzylalkohol | 0,300 kg |
| Oxynex LM | 0,030 kg |
| *Echinacea purpurea radix* CO₂-Extrakt | 0,030 kg |
| Magnesiumsulfat-Heptahydrat | 0,150 kg |
| Glycerol | 0,900 kg |
| Wasser, gereinigt | 18,840 kg |

**Hautmilch mit 0,02 % Echinacea purpurea radix CO₂-Extrakt:**

| | |
|---|---|
| Zinkstearat | 0,300 kg |
| Orange Peel Wax | 0,200 kg |
| Arlacel 1690 | 1,500 kg |
| Hexyldecyllaurat | 2,500 kg |
| Distelöl | 2,550 kg |
| Decyloleat | 1,500 kg |
| Isopropylmyristat | 1,200 kg |
| Benzylalkohol | 0,300 kg |
| Oxynex LM | 0,030 kg |
| *Echinacea purpurea radix* CO₂-Extrakt | 0,006 kg |
| Magnesiumsulfat-Heptahydrat | 0,150 kg |
| Glycerol | 0,900 kg |
| Wasser, gereinigt | 18,864 kg |

## Patentansprüche

1. Zusammensetzung enthaltend a) einen Extrakt aus *Echinacea* und b) Linolsäure und/oder Linolsäurederivate, wobei der Extrakt aus *Echinacea* von *Echinacea purpurea radix* stammt und die Linolsäurederivate in Form von Distelöl, Weizenkeimöl und/oder Rapsöl vorliegen.

2. Zusammensetzung nach Anspruch 1, **dadurch gekennzeichnet, dass** der Extrakt aus *Echinacea* ein lipophiler Extrakt ist.

3. Zusammensetzung nach Anspruch 1 und/oder Anspruch 2, **dadurch gekennzeichnet, dass** der Extrakt aus *Echinacea* ein CO₂-Extrakt ist.

4. Zusammensetzung nach einem der Ansprüche 1 - 3, **dadurch gekennzeichnet, dass** der Extrakt aus *Echinacea* einen Alkylamidgehalt zwischen 1 und 50%, bevorzugt zwischen 10 und 40% und besonders bevorzugt zwischen 15 und 30% hat.

5. Zusammensetzung nach einem der Ansprüche 1-4, **dadurch gekennzeichnet, dass** die Zusammensetzung die Bestandteile a) und b) in den nachfolgenden Gewichtsverhältnissen enthält: 1:1000 bis 1:1; bevorzugt 1:50 bis 1:10.

6. Zusammensetzung nach einem der Ansprüche 1-5, **dadurch gekennzeichnet, dass** 0,001 - 5 Gew.% *Echinacea*-Extrakt, bevorzugt 0,025 - 2 Gew.% *Echinacea*-Extrakt, bezogen auf das Gewicht der Gesamtzusammensetzung, und 0,001-10 Gew.% Linolsäure und/oder eine derartige Menge an Linolsäurederivaten, die 0,001-10 Gew.% Linolsäure entsprechen, bevorzugt 0,01-5 Gew.% Linolsäure und/oder eine derartige Menge an Linolsäurederivaten, die 0,01-5 Gew.% Linolsäure entsprechen, jeweils bezogen auf das Gewicht der Gesamtzusammensetzung, enthalten sind.

7. Zusammensetzung nach einem der Ansprüche 1-6, weiterhin umfassend einen oder mehrere kosmetische und/oder pharmazeutische Hilfsstoffe, bevorzugt in Form einer Emulsion.

8. Zusammensetzung nach einem der Ansprüche 1-7 zur Verwendung als Arzneimittel.

9. Verwendung der Zusammensetzung nach einem der Ansprüche 1-7 als Medizinprodukt.

10. Verwendung der Zusammensetzung nach einem der Ansprüche 1-7 als Kosmetikum.

11. Verwendung nach Anspruch 9 oder 10 zur Behandlung von trockener Haut.

12. Zusammensetzung nach einem der Ansprüche 1-7 zur Verwendung in der Behandlung von entzündlichen Zuständen der Haut und/oder Juckreiz und/oder irritierter Haut.

13. Zusammensetzung zur Verwendung nach Anspruch 12, wobei der entzündliche Zustand der Haut atopische Dermatitis ist.

## Claims

1. Composition containing a) an extract of *Echinacea* and b) linoleic acid and/or linoleic acid derivatives, wherein the extract of *Echinacea* originates from *Echinacea purpurea radix* and the linoleic acid derivatives are in the form of safflower oil, wheat germ oil and/or rapeseed oil.

2. Composition according to claim 1, **characterized in that** the extract of *Echinacea* is a lipophilic extract.

3. Composition according to claim 1 and/or claim 2, **characterized in that** the extract of *Echinacea* is a CO₂ extract.

4. Composition according to any of claims 1-3, **characterized in that** the extract of *Echinacea* has an alkylamide content of between 1 and 50%, preferably between 10 and 40% and particularly preferably between 15 and 30%.

5. Composition according to any of claims 1-4, **characterized in that** the composition contains components a) and b) in the following weight ratios: 1:1000 to 1:1; preferably 1:50 to 1:10.

6. Composition according to any of claims 1-5, **characterized in that** 0.001-5 wt.% *Echinacea* extract, preferably 0.025-2 wt.% *Echinacea* extract, based on the weight of the total composition, and 0.001-10 wt.% linoleic acid and/or such an amount of linoleic acid derivatives corresponding to 0.001-10 wt.% linoleic acid, preferably 0.01-5 wt.% linoleic acid and/or such an amount of linoleic acid derivatives corresponding to 0.01-5 wt.% linoleic acid, in each case based on the weight of the total composition, are contained.

7. Composition according to any of claims 1-6, further comprising one or more cosmetic and/or pharmaceutical auxiliaries, preferably in the form of an emulsion.

8. Composition according to any of claims 1-7 for use as a medicament.

9. Use of the composition according to any of claims 1-7 as a medicinal product.

10. Use of the composition according to any of claims 1-7 as a cosmetic.

11. Use according to claim 9 or 10 for treating dry skin.

12. Composition according to any of claims 1-7 for use in the treatment of inflammatory conditions of the skin and/or itching and/or irritated skin.

13. Composition for use according to claim 12, wherein the inflammatory condition of the skin is atopic dermatitis.

## Revendications

1. Composition contenant a) un extrait *d'Echinacea* et b) de l'acide linoléique et/ou des dérivés d'acide linoléique, l'extrait *d'Echinacea* provenant *d'Echinacea purpurea radix* et les dérivés d'acide linoléique étant présents sous forme d'huile de carthame, d'huile de germe de blé et/ou d'huile de colza.

2. Composition selon la revendication 1, **caractérisée en ce que** l'extrait *d'Echinacea* est un extrait lipophile.

3. Composition selon la revendication 1 et/ou la revendication 2, **caractérisée en ce que** l'extrait *d'Echinacea* est un extrait de CO₂.

4. Composition selon l'une des revendications 1 à 3, **caractérisée en ce que** l'extrait *d'Echinacea* possède une teneur en alkylamide comprise entre 1 et 50 %, de préférence entre 10 et 40 % et de manière particulièrement préférée entre 15 et 30 %.

5. Composition selon l'une des revendications 1 à 4, **caractérisée en ce que** la composition contient les constituants a) et b) dans les rapports pondéraux suivants : 1:1000 à 1:1 ; de préférence 1:50 à 1:10.

6. Composition selon l'une des revendications 1 à 5, **caractérisée en ce qu'**elle contient 0,001 à 5 % en poids d'extrait *d'Echinacea,* de préférence 0,025 à 2 % en poids d'extrait *d'Echinacea,* par rapport au poids de la composition totale, et 0,001 à 10 % en poids d'acide linoléique et/ou une quantité de dérivés d'acide linoléique correspondant à 0,001 à 10% en poids d'acide linoléique, de préférence 0,01 à 5% en poids d'acide linoléique et/ou une quantité de dérivés d'acide linoléique correspondant à 0,01 à 5 % en poids d'acide linoléique, respectivement par rapport au poids de la composition totale.

7. Composition selon l'une des revendications 1 à 6, comprenant en outre un ou plusieurs auxiliaires cosmétiques et/ou pharmaceutiques, de préférence sous forme d'une émulsion.

8. Composition selon l'une des revendications 1 à 7 pour l'utilisation comme médicament.

9. Utilisation de la composition selon l'une des revendications 1 à 7 comme produit médical.

10. Utilisation de la composition selon l'une des revendications 1 à 7 comme produit cosmétique.

11. Utilisation selon la revendication 9 ou 10 pour le traitement de peaux sèches.

12. Composition selon l'une des revendications 1 à 7 pour l'utilisation dans le traitement des états inflammatoires de la peau et/ou des démangeaisons et/ou des peaux irritées.

13. Composition pour l'utilisation selon la revendication 12, l'état inflammatoire de la peau étant la dermatite atopique.
